# EUROPEAN PATENT SPECIFICATION

(11) **EP 4 337 153 B1**
(45) Date of publication and mention of the grant of the patent: **17.12.2025**
(21) Application number: 22727656.5
(22) Date of filing: 12.05.2022
(51) Int. Cl.: A61F 13/02, A61F 13/00

(54) **ANTIMICROBIAL PUNCTURE SITE PATCH WITH POST INJECTION SAFETY SEALING**
ANTIMIKROBIELLES PUNKTIONSSTELLENPFLASTER MIT SICHERHEITSABDICHTUNG NACH DER INJEKTION
TIMBRE ANTIMICROBIEN POUR SITE DE PERFORATION AVEC SCELLEMENT DE SÉCURITÉ POST-INJECTION

(30) Priority: 13.05.2021 US 202163188166 P
(43) Date of publication of application: 20.03.2024
(73) Proprietor: Injection Safety Industries, Llc, Bowling Green, Kentucky 42104 (US)
(72) Inventor: HARRIS, Mark, Bowling Green, Kentucky 42104 (US); WATSON, Robert, deceased (US)
(74) Representative: SSM Sandmair
(86) International application number: PCT/US2022/029061
(87) International publication number: WO 2022/241161

(56) References cited:
- WO-A2-2007/106068
- US-A- 5 160 315
- US-B1- 6 685 682

## Description

It is widely recognized that bloodborne pathogens are an important method of transmission of infectious diseases. Health care professionals, in particular, are in danger from such exposure because of the need to perform functions that directly expose the healthcare professional to blood, such as puncturing a patient's skin with a needle to make an injection or to withdraw blood. Typically, when skin is to be punctured, the target area of the skin is wiped with a disinfectant (e.g. an alcohol wipe) and the needle (which may be further attached to a syringe) is injected through the skin subcutaneously or intramuscularly. After the skin has been punctured, the needle is withdrawn and a pad of gauze or cotton is placed on the puncture wound to absorb any blood which may emanate therefrom. The practitioner may come into contact with the blood at several points during this procedure; for example, when the needle first punctures the skin, when the needle is removed from the skin but before the pad is positioned over the puncture wound, or after the pad is applied over the puncture site because blood may ooze from or seep through the pad. Furthermore, gloves may fail, and such a failure puts a healthcare professional at additional risk for exposure to pathogens. Thus, it may be desirable to have a protective patch for use during injections that incorporates a microbicidal, microbiostatic, or/and anti-viral agent to kill and/or reduce the infectivity of microbial organisms (e.g. viruses, bacterial, fungi, etc.) that may have been exposed as a result of the puncture to the skin.

However, the risk of exposure to bloodborne or other pathogens does not end with the administration of a vaccination or other puncture wound to the skin. Blood and/or other components may be contained on the gauze, bandage, etc. placed on the puncture wound. These items subsequently require safe disposal. Additionally, the increase in popularity of drive thru vaccination clinics, for example for flu vaccine or the SARS-coV-2 vaccine means gauze, bandages, or the like may be removed and discarded in areas such as patients' homes, restaurants, bathrooms, parking lots, or elsewhere where other individuals may be exposed to bloodborne pathogens.

WO 2007/106068 A2 discloses an elastomeric puncture site patch for adhering to skin and antiseptically covering the skin at an area where a sharp object such as a syringe needle or intravenous catheter is to be inserted. The patch is comprised of an elastomeric transparent self-sealing membrane and a spacer having an aperture. The spacer has an adhesive film on the surface opposite the membrane for adhering the patch to the skin. When the patch is applied to an area of the skin, a chamber is formed between the skin and the membrane and bounded by the spacer. In use, the skin is antiseptically cleaned and the patch applied. The syringe needle or similar device punctures the membrane and the skin. At the end of the procedure the needle is withdrawn and any blood remaining on the outside of the needle or oozing from the wound is trapped in the chamber, thus preventing contamination of the area beyond the chamber. A method of manufacturing the puncture site patch is also provided.

### Summary

The invention is set out in appended claim 1. The dependent claims describe advantageous embodiments. The herein-described embodiments address these and other problems associated with the art by providing a puncture site patch with post-injection safety sealing. Therefore, consistent with one aspect of the invention, a puncture site patch, that includes: an elastomeric, self-sealing membrane with an exterior face and an interior face; a spacer including an outer face and an inner face, where the outer face of the spacer is coupled to the interior face of the elastomeric, self-sealing membrane and where the spacer also includes an aperture that forms a cavity defined by the interior face of the elastomeric, self-sealing membrane, spacer, and skin when applied to a user; an adhesive film to adhere to skin, that is located on the inner face of the spacer; at least one elongated tab that can move between a first position and a second position; where when in the first position the at least one elongated tab extends outward from a periphery of the spacer; and where when in the second position the puncture site patch is folded so that the aperture is sealed closed by the at least one elongated tab.

In some implementations, the at least one elongated tab is a first elongated tab and the puncture site patch further includes a second elongated tab, where the first and second elongated tabs each extend outward from the periphery of the spacer and are disposed directly opposing each other. In some such implementations, when in the second position the first and second elongated tabs substantially overlap each other.

In some implementations, the puncture site patch additionally includes an axis running through a center of the aperture which defines a first and second side of the spacer, and where when in the second position the puncture site patch is folded along the axis such that the first and second sides of the spacer substantially overlap each other and the at least one elongated tab is folded over the substantially overlapping first and second sides of the spacer to seal the first and second sides of the spacer closed.

In some implementations, when in the second position the at least one elongated tab is folded over the aperture to seal the cavity closed.

In some implementations, the puncture site patch additionally includes a release paper in contact with and completely covering the adhesive film, where the release paper is removed prior to application to skin.

In some implementations, the at least one elongated tab further included deadened adhesive. In other implementations, the elastomeric, self-sealing membrane is substantially transparent.

The elastomeric, self-sealing membrane further includes an antimicrobial agent. According to the invention the antimicrobial agent is incorporated into the elastomeric, self-sealing membrane during extrusion. In some implementations, the antimicrobial agent is a plurality of silver nanoparticles. In other implementations, the antimicrobial agent is a carvacrol agent incorporated into the elastomeric, self-sealing, membrane at a concentration of about 0.01% to about 0.001%.

These and other advantages and features, which characterize the invention, are set forth in the claims annexed hereto and forming a further part hereof. However, for a better understanding of the invention, and of the advantages and objectives attained through its use, reference should be made to the Drawings, and to the accompanying descriptive matter, in which there is described example embodiments of the invention. This summary is merely provided to introduce a selection of concepts that are further described below in the detailed description, and is not intended to identify key or essential features of the claimed subject matter, nor is it intended to be used as an aid in limiting the scope of the claimed subject matter.

### Brief Description of the Drawings

FIG. 1 is a top view of the antimicrobial puncture site patch consistent with one embodiment described herein.
FIG. 2 is an exploded perspective view of the patch of FIG. 1.
FIG. 3A,and FIG. 3B are a cross-sectional views of the patch of FIG. 1. FIG. 3A is a cross-sectional view of the patch of FIG. 1 in an unused configuration; FIG. 3B is a cross-sectional view of the patch of FIG. 1 in a disposal configuration.
FIG. 4A, and FIG. 4B are perspective views of the patch of FIG. 1 positioned on a patient's arm.
FIG. 5 is a top view of an antimicrobial puncture site patch consistent with another embodiment described herein.
FIG. 6 is an exploded perspective view of the patch of FIG. 5.
FIG. 7A, FIG. 7B, FIG. 7C are a cross-sectional views of the patch of FIG. 5. FIG. 7A is a cross-sectional view of the patch of FIG. 5 in an unused configuration; FIG. 7B is a cross-sectional view of the patch of FIG. 5 in a first disposal configuration; FIG. 7C is a cross-sectional view of the patch of FIG. 5 in a second disposal configuration.
FIGS. 8A, and FIG. 8B are perspective views of the patch of FIG. 5 positioned on a patient's arm.
FIG. 9 is a top view of the patch of FIG. 5 in a disposal configuration.
FIG. 10 is a perspective view of a package consistent with some embodiments described herein for containing the antimicrobial puncture site patch of FIG. 5.
FIG. 11 is a schematic view of a process by which an antimicrobial puncture site patch can be made.

### Detailed Description

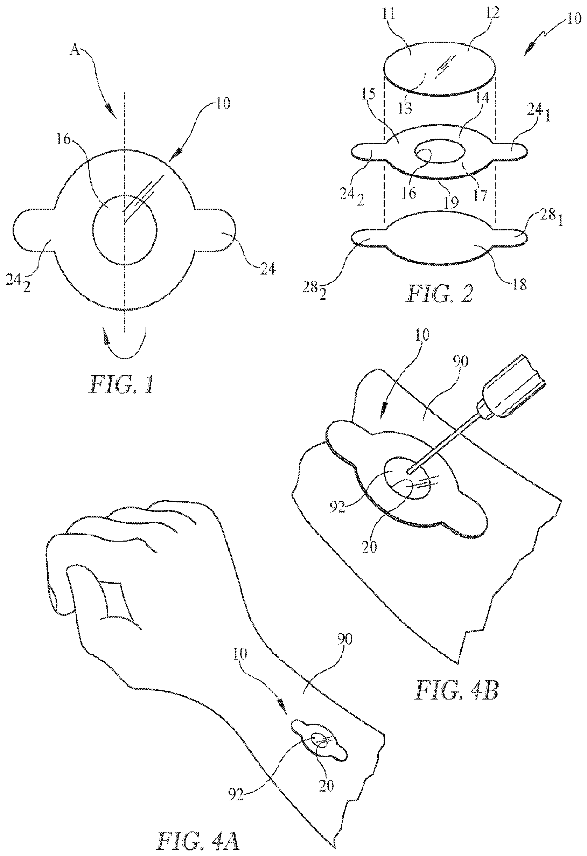

Figures 1-3 illustrate an antimicrobial puncture site patch device 10 (hereinafter "the antimicrobial patch", "the patch", etc.). The patch 10 includes a membrane 12 and a spacer 14, where the spacer 14 further includes an aperture 16. In the illustrated embodiment, the patch device 10 is generally circular; however, this is not intended to be limiting, as other shapes may be used including, but not limited to, square-shaped, rectangular, oval-shaped, triangular, and so on. In some instances, for example as illustrated in Figure 2, the patch 10 may also, optionally, include a protective sheet or release paper 18. This protective sheet or release paper 18 may be used to protect an adhesive film 26 on the spacer 14 (as will be described in detail herein) until the patch 10 is to be applied to a patient's skin. In some instances, the protective sheet or release paper 18 may be a similar size and shape as the spacer 14, or, in other instances, the protective sheet or release paper 18 may be slightly larger than the spacer 14.

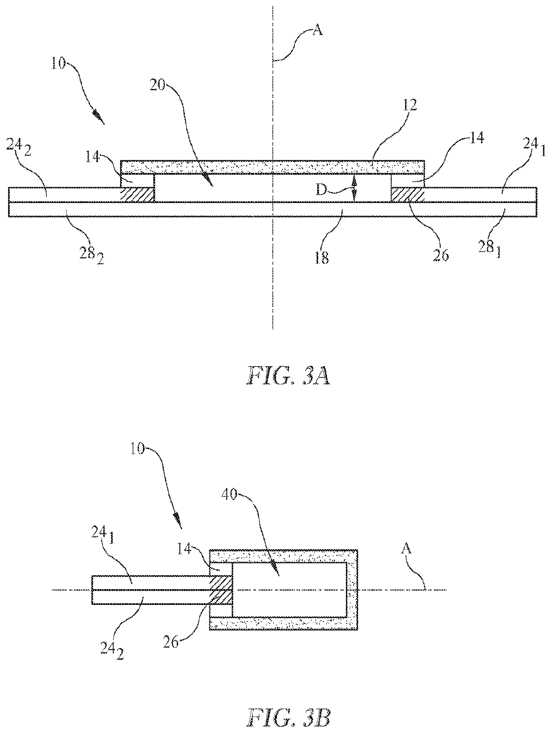

The protective sheet or release paper 18, in some instances, may also include a first release tab 28₁ and a second release tab 28₂, or other surface, to allow for a user to easily remove of the release paper 18 from the membrane 12 and spacer 14, which exposes the adhesive film 26. In some instances, the patch 10 may also include a first pull tab 24₁ and second pull tab 24₂ that may protrude from the spacer 14. The first and second pull tabs 24_{1, 2} may be positioned directly across the aperture 16 from each other, such that (as described in detail herein) when the antimicrobial puncture site patch device 10 is folded along an axis A, first and second pull tabs 24_{1, 2} may contact each other (see e.g. Fig. 3B) to seal the patch 10. As will be discussed herein, folding the first and second pull tabs 24_{1, 2} in this manner may also seal the interior of the patch 10, so as to prevent contact with and the spread of pathogens in the post injection environment.

Furthermore, these pull tabs 24_{1, 2} may facilitate the removal of the patch 10, as they may not include adhesive. In some instances, only one of the pull tabs 24_{1, 2} may be without adhesive film 26; while in other instances, both pull tabs 24_{1, 2} may lack adhesive. This may, in some instances, allow a user to grasp the portion of one or both of the pull tabs 24_{1, 2}, particularly where the adhesive film 26 may be absent, in order to remove the patch 10 from the skin.

The membrane 12 of the antimicrobial patch 10 may be an extruded elastomeric material. In some instances, this elastomeric material may be sterile, self-sealing, non-coring, non-latex and FDA-approved for direct contact with human or animal skin. Due to the self-sealing and non-coring nature of the elastomeric material, needles (of various sizes) used for injections through the membrane 12 may be wiped clean of blood on the withdrawal of the needle through the membrane 12. The membrane 12 may also, in some instances, be sufficiently transparent that the practitioner may observe the patient's skin 90 when the patch 10 is positioned over the intended injection site 92. The physical structure of the membrane 12 includes an exterior face 11 and an interior face 13, the latter of which faces toward the patient's skin 90 when the antimicrobial patch 10 is applied. Further, the membrane material may be selected such that it is stable and essentially unaffected chemically or structurally, and therefore will not degrade if cleaned, disinfected and/or sterilized with agents such as ethyl alcohol, betadine, or similar common disinfectants. The membrane 12 may have any convenient shape that sufficiently covers the spacer 14. In some embodiments, the exterior peripheries of the membrane 12 and spacer 14 may be substantially the same; while in other embodiments the peripheries may vary.

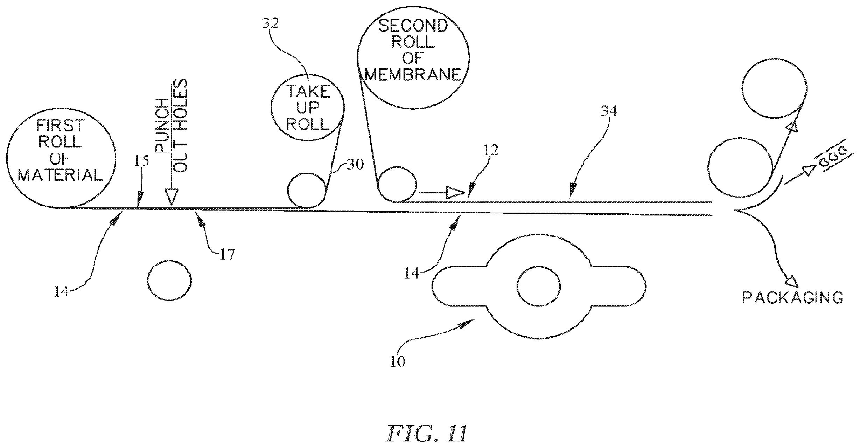

The membrane 12 may further be infused with one or more antimicrobial agents. This infusion may be, in some instances, completed during the curing process of the elastomeric material (i.e. during the heating and extrusion of the polymer), as is further described with reference to Figure 11; this incorporation may be via extrusion or co-extrusion. There are a variety of antimicrobial agents both natural and synthesized. For example, some of these antimicrobial agents may be derived from various plants or herbs. Some examples of such antimicrobial herbs include, but are not limited to, components of oregano, sage, basil, fennel, garlic, lemon balm, peppermint, rosemary, Echinacea, sambucus, licorice, astragalus, ginger, ginseng, and/or dandelion. In other embodiments, synthetic or lab produced antimicrobial agents may also be used, either alone or in combination with each other or any naturally occurring herbaceous antimicrobial agents. In still other instances, one or more metals may be used as the antimicrobial agent, for example silver or copper. These antimicrobial agents may provide varying mechanisms of action for their respective antimicrobial activity. These agents may be effective against bacteria, viruses, and/or fungi, and may also be bactericidal, viricidal, and/or fungicidal and/or may be bacteriostatic, virustatic, and/or fungistatic. Any of the these above referenced agents may be incorporated into the elastomeric membrane 12; however, an important consideration in selecting one or more microbial agents is if the agent will remain active after being exposed to the conditions (e.g. heat, pressure, etc.) of extrusion of the elastomeric membrane 12, as described with reference to Fig. 11. According to the invention the antimicrobial is incorporated into elastomeric membrane 12 through extrusion.

As a non-limiting example, carvacrol, which is a phenolic monoterpenoid of oregano (*Origanum vulgare*), may be incorporated into the elastomeric membrane 12 of the antimicrobial patch 10. Test tube studies have illustrated that carvacrol may reduce the activity of murine norovirus after about 15 minutes of exposure. Furthermore, carvacrol exhibits antiviral activity against a wide range of viruses, including herpes simplex viruses, rotavirus, respiratory syncytial virus, etc. Additionally, due to its phenolic structure carcacrol is expected to be effective against coronaviruses, including SARS-coV-2. The inhibitory action of carvacrol may be related to its hydrophobicity and lipophilic tendencies, as these characteristics result in an affinity for cell membranes and insertions therein that change the physical and chemical properties of the cell membrane and alter stability of the membrane. In some instances, the carvacrol agents may be incorporated into the elastomeric membrane 12 during the extrusion and/or curing process at a concentration of about 0.01% to about 0.001%.

As another non-limiting example, a metal antimicrobial agent may be incorporated into or coated on to the elastomeric membrane 12 of the antimicrobial patch 10. These metal antimicrobial agents may, for example, cause cell damage to the microorganisms as a result of reactive oxygen species (ROS), other oxidative processes, an exchange of structural metals, and/or the destruction of iron-sulfur clusters. In some instances, silver nanoparticles or copper nanoparticles may be used as the metal antimicrobial. These metal nanoparticles may be included in the extrusion of the polymer forming the membrane 12 and/or may be coated onto the membrane 12 surface. In some instances, the silver nanoparticles may be formed *in situ,* meaning that the polymer(s) utilized for the formation of the elastomeric membrane may be combined with a metal salt and reducing agent and the metal nanoparticle is formed during the combination. In other instances, the pre-synthesized metal nanoparticles may be dispersed with the polymer medium. In some instances, the silver may be incorporated into the membrane 12 at a concentration of about 0.1% to about 4%. In other instance, the silver may be incorporated into the membrane at a concentration of about 2.5%, at this concentration studies have shown the silver to be effective against multiple species of microorganisms. In one example, a silver-zeolite based antimicrobial is incorporated into the membrane.

The spacer 14 is a material having an outer face 15 and an inner face 17. The spacer 14 can be composed of any material that can be sterilized and that can be used in direct contact with human skin, such as nylon, polyurethane, polyethylene, polypropylene, isoprene, cotton, linen, or combinations thereof. Optionally, the material for the spacer 14 may be selected on the basis of its ability to absorb blood and bodily fluids, although these absorptive properties are not required. In some instances, where an absorbent material is used it may be desirable, although not required, for the absorbent material be somewhat resistant to wicking so as to minimize the risk of blood wicking from the cavity 20 to an outside edge 19 of the spacer 14. In some instances, one or more additives may be incorporated into the extrusion layer in order to add absorbency. The spacer 14 is secured at or on its outer face 15 to the interior face 13 of the membrane 12. A variety of means known in the art can be used to secure the spacer 14 to the membrane 12, such as glue, hot melt adhesive, pressure sensitive adhesive, thermally sensitive adhesive, chemical bonding, acrylic cement, radio frequency welding, ultrasonic welding or a combination thereof, or any other means well known in the art. However, an important consideration in the selection of the securing means for the spacer is what if any interaction the selected securing means may have on the efficacy of the selected antimicrobial agent. The spacer 14 may, in some instances, be positioned on the membrane 12, such that the membrane 12 covers the entire outer face 15 of the spacer 14. The inner face 17 of the spacer 14 is coated with an adhesive film 26 for holding the patch 10 against a patient's skin 90. Although a variety of adhesives may perform the desired function, it is preferable that the adhesive utilized be an FDA-approved material due to the adhesive's direct contact with human skin.

This spacer 14 may additionally include an aperture 16. In some instances, this aperture may be substantially centrally located. The aperture 16 has dimensions adequate to allow a practitioner to insert a needle through the membrane 12 and the aperture 16 without penetrating or damaging any part of the spacer 14. When the antimicrobial patch 10 is applied to a patient's skin 90, the aperture 16 bounded by the skin 90, the membrane 12, and the spacer 14 forms a cavity 20, through which a needle passes during an injection or blood withdrawal procedure. Any blood released from the skin from the puncture may pool in the cavity 20. In some instances, the blood may dry within the cavity 20, or, in other instances, it may be absorbed by the spacer 14. The spacer may include, or be constructed of, an absorbent material, and may optionally include a coagulating agent. In a non-limiting example, the spacer 14 may be constructed from polyethylene and be coated with oxidized regenerated cellulose or any other suitable coagulant generally known in the art to coagulate blood. In some instances, the aperture 16 is about 15 mm (0.591 in) in diameter are such that the cavity 20 thereby formed may contain up to about 0.3 cc of bodily fluids. It is to be understood that the dimensions of the aperture 16 and antimicrobial patch are not limited to those described herein and may vary based on the desired use of the antimicrobial patch 10. It is to be further understood that the volume the formed cavity may contain may vary as the dimensions of the aperture vary.

Referring now to FIGS. 4A and 4B, the antimicrobial patch 10 is illustrated as applied to a patient's skin 90 over an intended puncture site 92, for example an injection site. When the patch 10 is attached to the skin 90, a cavity 20 forms between the skin 90 and the membrane 12 bounded by the spacer 14. This cavity 20 may, in some instances, have a depth D. The antimicrobial patch 10 may be secured and held directly against the patient's skin 90 along any portion of the inner face 17 of the spacer 14 via the adhesive film 26. This secure attachment may be beneficial to prevent blood leakage and therefore potential spread of blood-borne pathogens, it may also be problematic for removing the antimicrobial patch 10 from the skin after use. To facilitate removal, the antimicrobial patch 10 may further include a first and second pull tab 24_{1, 2} that protrude from the spacer 14 on opposing sides. In some instances, these pull tabs 24_{1, 2} may be unitary with the spacer 14. As discussed previously, the first and/or second pull tab 24_{1, 2} may or may not include adhesive on the inner face 26, and in some instances may only partially contain adhesive. In other instances, the adhesive may be deadened over part or the entirety of the first and/or second pull tabs 24_{1, 2}. so that either a practitioner or patient may easily grab a portion of the one or both of the pull tabs 24_{1, 2}, for example a portion without adhesive, to remove the antimicrobial patch 10 from the patient's skin.

Now referring specifically to Figs. 3A-B, cross-sectional views of the antimicrobial patch 10 are provided in a first, unused configuration (Fig. 3A) and in a second, disposal configuration (Fig. 3B). As illustrated in Fig. 3A, the starting position of the first and second pull tabs 24_{1, 2} are extending directly opposed each other outward from the spacer 14. At some point subsequent the injection or other puncture through the membrane 12 and skin, a patient, caregiver, or healthcare provider may desire to remove the antimicrobial patch 10. At such a point, the patient, caregiver, etc. may easily grab a portion of the one or both of the pull tabs 24_{1, 2} and fold the antimicrobial patch 10 along a center axis A, so as to ensuring the aperture 16 is completely covered. Once folded the first and second pull tabs 24_{1, 2} may substantially overlap each other. In some instances, there may be an indentation, marking, or other guide to facilitate the patient or caregiver in knowing precisely where to fold the patch 10. The existing adhesive film 26 on the antimicrobial patch 10 is used to create a sealed enclosure 40 to lock in and prevent leakage of bodily fluids, pathogens, etc. while simultaneously exposing the bodily fluids, and any subsequent blood-borne pathogens to the embedded antimicrobial agent. In some instances, the adhesive film 26 extends on to a part (or the entirety of) one or both of the pull tabs 24_{1, 2} so that they may further seal the patch 10. Once trapped in the sealed enclosure 40 or collection cavity, any blood, viral particles, bacteria, or the like contained therein may be in contact with the antimicrobial membrane 12; contact with this antimicrobial membrane 12 for about 15 minutes or longer may result in the inactivation of any viral particles, making disposal of the patch 10 safe.

As mentioned previously, there has been an increase in popularity of drive thru vaccination clinics, or other high throughput vaccination clinics. The increase in such high throughput clinics increases the likelihood that any bandage, such as the antimicrobial puncture site patch 10, used would likely be removed and discarded in areas such as patients' homes or elsewhere where other individuals may be exposed to blood-borne pathogens. Once the antimicrobial patch 10 is in the disposal configuration illustrated in Fig. 3B, the patch 10 may be safely disposed of with minimal risk of exposing others to bodily fluids or pathogens that may be contained therein.

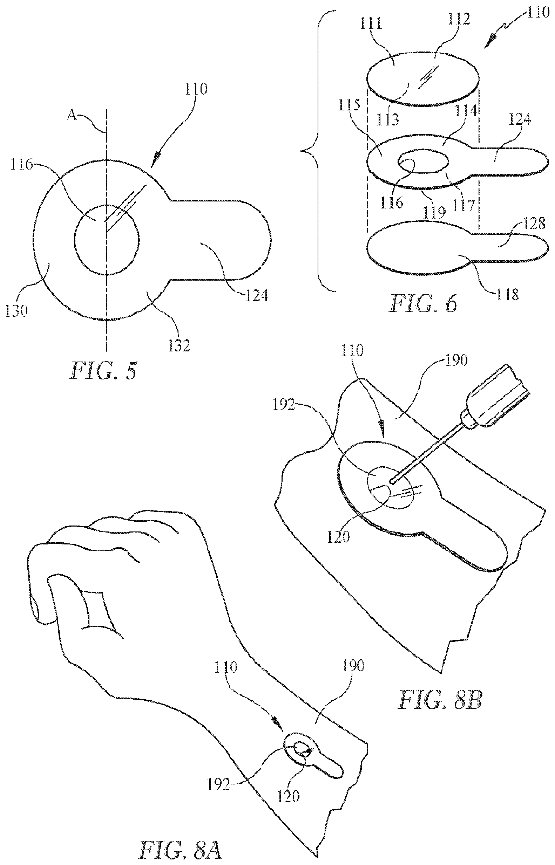

Figures 5-6, 7A-C, 8A-B, and 9-10 illustrate another embodiment of an antimicrobial puncture site patch 110. Similar to the embodiments previously discussed, the patch 110 may also include a membrane 112 and a spacer 114 with an aperture 116. Similar to the previous embodiments discussed herein the patch 110 is substantially circular; however, as described with the previous embodiment, this is not intended to be limiting. The patch 110 may also, optionally, include a protective sheet or release paper 118 to protect an adhesive film 126 on the spacer 114. The release paper 118 may be a similar size and shape as the spacer 114, but is not so limited, as it may also be slightly larger than the spacer 114. The release paper 118 may also include a release tab 128, or other surface, to allow for easy removal of the release paper 118 from the membrane 112 and spacer 114. The patch 110 may also include an elongated pull tab 124 that may protrude from the spacer 114. This elongated pull tab 124 may facilitate the removal of the patch 110 from a patient.

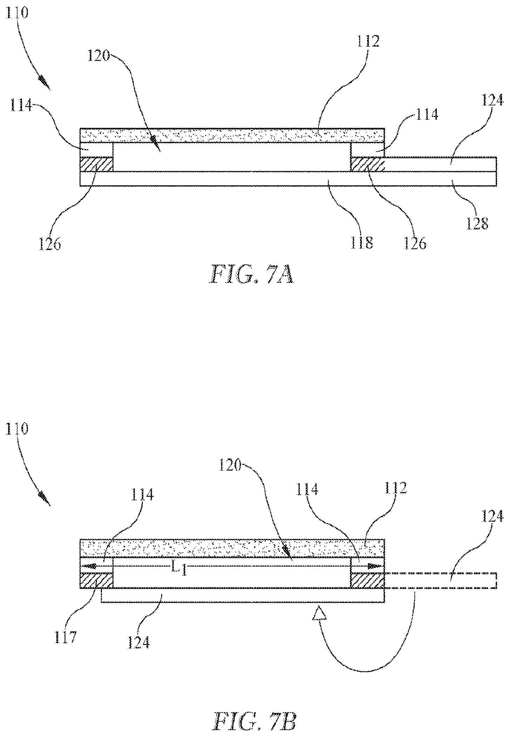

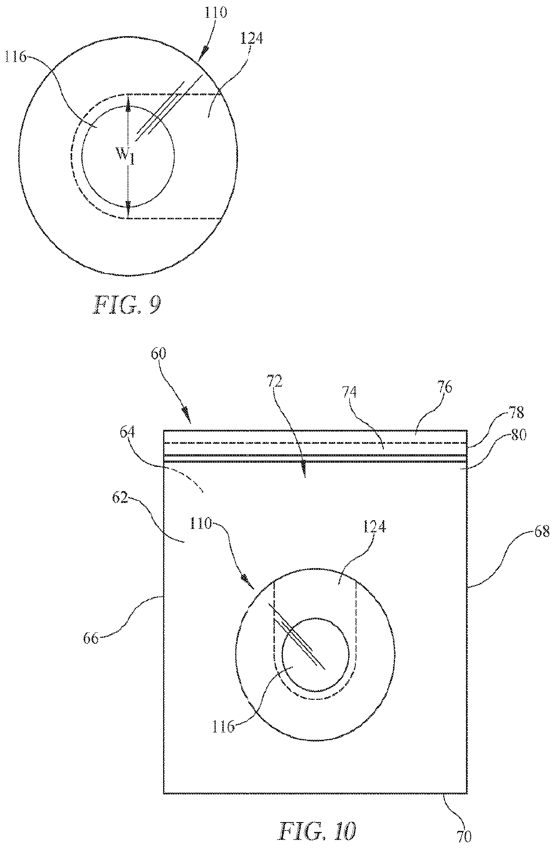

As illustrated in Fig. 7B, in some instances, this elongated pull tab 124 may be large enough to be folded over on the remainder of the adhesive film 126 (e.g. on the elongated pull tab 124) to completely enclose the cavity 120 during the disposal process (see also Fig. 9). Folding the elongated pull tab 124 over on the remainder of the adhesive film 126 and enclosing the cavity 120 ensures the antimicrobial agent on the membrane remains in contact with any contaminated blood on the adhesive layer and seals the cavity 120 to prevent any contact with the bodily fluids and/or pathogens contained therein.

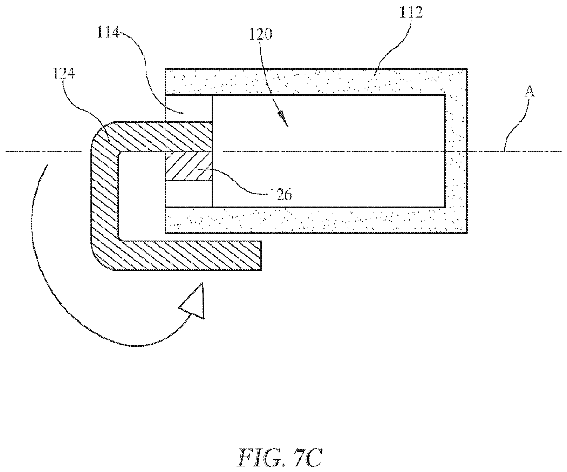

In other instances, as illustrated in Fig. 7C, the antimicrobial patch 110 may be folded approximately in half along a center axis A, which defines a first side of the spacer 130 and a second side of the spacer 132. When the antimicrobial patch 110 is folded along the central axis A, the first side of the spacer 130 and second side of the spacer 132 may be substantially overlapped, so as to ensure the cavity 120 is completely enclosed, forming closed patch 110. In some instances, there may be an indentation, marking, or other guide to facilitate the patient or caregiver in knowing precisely where to fold the patch 110. The elongated pull tab 124 may then be folded over the closed patch 110, and the adhesive film 126 on the elongated tab may be used to further seal the patch 110 in the closed position.

The membrane 112 of the antimicrobial patch 110 may be an elastomeric material similar to any to the elastomeric material described with reference to the previous embodiments. As described previously it may be advantageous, in some instances, for the membrane 112 to be sufficiently transparent that the practitioner may observe the patient's skin 190 when the patch 110 is positioned over the intended injection site 192. In some instances, this transparency may be defined by a total transmittance measurement, haze measurement, clarity measurement, optical density, or any other known measures. Similar to the previously described membrane, membrane 112 may also include an exterior face 111 and an interior face 113. As described previously, the membrane 112 may further be infused with one or more antimicrobial agents.

The spacer 114 includes an outer face 115 and an inner face 117, and is constructed of a material that may be placed in direct contract with human skin, such as nylon, polyurethane, polyethylene, polypropylene, isoprene, cotton, linen, or combinations thereof. The material used in constructing the spacer 114 may also, in some instances, be selected on the basis of its ability to absorb various bodily fluids. Where such an absorbent material is used, the absorbent material may, in some instances, be somewhat resistant to wicking so as to minimize the risk of blood wicking from the cavity 120 to an outside edge 119 of the spacer 114. Similar to previously described embodiments, the spacer 114 may be secured at or on its outer face 115 to the interior face 113 of the membrane 112 by a variety of means known in the art; however, as mentioned previously an important factor in selecting the method of securing the spacer 114 is what, if any, interaction the selected securing method may have on the efficacy of the selected antimicrobial agent. The inner face 117 of the spacer 114 may be coated with an adhesive film 126 to facilitate holding the patch 110 against a patient's skin 190. In some instances, the spacer 114 may additionally include a substantially centrally located aperture 116 that has dimensions adequate for a practitioner to insert a needle through the membrane 112 without penetrating or damaging any part of the spacer 114. Similar to the previous embodiments, once applied the patch is bounded by the skin 190, the membrane 112, and the spacer 114 thereby forming a cavity 120. Any bodily fluids released from the skin from the puncture may pool in this cavity 120.

Referring now to FIGS. 8A and 8B, the antimicrobial patch 110 is illustrated as applied to a patient's skin 190 over an intended puncture site 192, as described this forms a cavity 120 between the skin 190 and the membrane 112 bounded by the spacer 114. This cavity 120 may, in some instances, have a depth D generally equal to the width W of the spacer 114 plus adhesive film 126. The elongated pull tab 124 that protrudes from the spacer 114 may facilitate removal of the antimicrobial patch 110. In some instances, the elongated pull tab 124 may not contain an adhesive film; while in other instances, the elongated pull tab 124 may only partially contain an adhesive film 126, such that a portion of the elongated pull tab 124 remains free of adhesive. The elongated pull tab 124 or the adhesive-free portion thereof may facilitate a practitioner or patient in grasping the elongated pull tab 124. In some instances, elongated pull tab 124 may be long and wide enough to be capable of folding over the entirety of the aperture 116 during the disposal process.

Referring now to FIGS. 7A-B, where cross-sectional views of the antimicrobial patch 110 in an unused configuration (Fig. 7A) and in a disposal position (Fig. 7B) are illustrated. In the disposal position, the elongated pull tab 124 is folded over the aperture 116, this movement is indicated by the arrow. The original, starting position of the elongated pull tab 124 (e.g. extending outward from the spacer 114), is illustrated by the broken line in Fig. 7B. In some instances, the elongated pull tab 124 is long enough so as to cover substantially all of the adhesive film 126, which would require the length of the elongated pull tab to be about that of L1. In other instances, the elongated pull tab 124 may only be long enough so as to cover the entirety of the aperture 116. The illustrated length is not to be construed as limiting, as the length of the elongated pull tab 124 may be any length sufficient completely cover the aperture 116. Similarly, the width of the elongated pull tab 124 may also be any length sufficient completely cover the aperture 116. A non-limiting example of the width W1 of the elongated pull tab 124 is illustrated in Fig. 9. Ensuring the aperture 116 is completely covered when folding the elongated pull tab 124 over, also ensures antimicrobial agent on the membrane 112 remains in contact with any contaminated blood on the adhesive film 126 and seals the cavity 120 to prevent any contact with the bodily fluids and/or pathogens contained therein.

The increased likelihood that any bandage, such as antimicrobial puncture site patch 110, used in conjunction with vaccination or other punctures may be removed and discarded in areas such as patients' homes or elsewhere necessitates a disposal solution for minimizing the risk of spreading blood-borne pathogens via the contaminated bandage. In some embodiments, the antimicrobial patch 110 may be sterilely packaged in a reusable container 60 that may be used for disposal of the patch 10 following removal from the patient. A non-limiting example of the such a reusable container 60 is illustrated in Figure 10.

As illustrated in Figure 10, the reusable container 60 may be formed into the general shape of a rectangle or square sufficient to hold a single antimicrobial patch 110; however, this is not intended to be limiting, as any shape bad may be used so long as it is sufficient to contain an antimicrobial patch 110. The reusable container 60 may generally be in the form of a bag, such that it is constructed of one or more pieces of a polymer, plastic, or the like sealed to together. In some instances, the reusable container 60 may include a first polymer sheet 62 and a second polymer sheet 64, where the two sheets 62, 64 are sealed together forming a first edge 66, a second edge 68, and bottom 70 portion of the reusable container 60. The first edge 66, second edge 68, and bottom 70 portion collective form a cavity 72 between the first and second sheets 62, 64. The reusable container 60 may further include an opening 74, through which the antimicrobial patch 110 is placed into the cavity 72.

In some instances, the reusable container 60 may further include a tear line 76 with notch (or perforation) 78 at one or both edges 66, 68 disposed proximate a top portion of the reusable container 60. This notch(es) may facilitate a user, for example a healthcare provider about to administer a vaccine, in opening the container 60. In some instances, the reusable container 60 may further contain a closure means 80 to allow for reinsertion of the antimicrobial patch following use and reseal the reusable container 60. One non-limiting example of such a closure 60 may be a closure member on a first sheet 62 that cooperates with a complementary closure element on a second sheet 64, as is typical of conventional "zip" type closures. Another non-limiting example of the closure may be an adhesive strip and corresponding protective cover on the exterior of one sheet 62, 64 of the container 60.

In some instances, the reusable container 60 may further include an antimicrobial agent. In some instances, only the portions of the reusable container 60 that may come into contact with the antimicrobial patch 110, such as those contained within the cavity 72, are infused or coated with the antimicrobial agent. In other instances, the entire reusable container 60 may be infused or coated with an antimicrobial agent. This infusion may, for example, be completed during the production and/or curing process for the polymer or plastic used to construct the container 60. As discussed previously, there may be a variety of antimicrobial agents both natural and synthesized that may be incorporated into the reusable container 60. As discussed with reference to antimicrobial patch 10, 110 one non-limiting example of such an antimicrobial agent may be carvacrol, which is a phenolic monoterpenoid of oregano (*Origanum vulgare*)*.* Carvacrol may be incorporated into the polymer or plastic of which the reusable container 60 is constructed. In some instances, the carvacrol agents may be incorporated into the elastomeric membrane 12 during the extrusion and/or curing process at a concentration of about 0.01% to about 0.001.

Although described and illustrated using antimicrobial patch 110, it is to be understood that the reusable container 60 described and illustrated herein is not so limited, so a disposal container may be used with antimicrobial patch 10, or any other embodiment of antimicrobial patch contemplated herein.

Furthermore, although primarily discussed herein with reference to a vaccination, this is not intended to be limited. The various embodiments of antimicrobial patches 10, 110 consistent with the embodiments described herein may also be used for insertion of a catheter into a patient, needle biopsies, joint injections or aspirations, or any other instances where a patient's skin may be punctured by a sharp, needle-like object. Furthermore, the cavity can accommodate bodily fluids other than blood, such as an aspirate, etc.

Referring now to Figure 11, an exemplary method for the continuous production process for the generation of an antimicrobial patch described herein is illustrated. The creation of an antimicrobial patch 10 may include creating apertures 16 at one or more predetermined positions along a sheet of spacer material 14 (e.g. as nylon, polyurethane, polyethylene, polypropylene, isoprene, cotton, linen). As discussed previously, this aperture 16, may be substantially circular, but is not so limited. In some instances, this spacer material 14 may be pretreated with an adhesive film 26 on both faces 15, 17. In other instances, it may be necessary to apply an adhesive film 26 to the spacer material 14. The outer face 15 of the spacer material 14 may also include a sheet of protective paper 30 disposed thereon. This sheet may function to protect the outer face 15 prior to the application of the membrane material 12 to the outer face 15. The protective paper 30 may be removed via a take-up roll 32 as the punched spacer material continues its forward motion. Similarly, a sheet of release paper 18 may be disposed on the inner face 17 to protect the adhesive film 26 prior to use. A sheet of elastomeric membrane material 12 may then be secured to the outer face 15. The membrane 12, spacer member 14, release paper 18 combination may then be die cut 34 into the desired configuration.

The sheet(s) of the elastomeric membrane, infused with the antimicrobial agent as described herein, may be separately formed prior to incorporation into the process described with reference to Figure 11. The elastomeric membrane 12 is a film extruded on standard plastics-processing equipment by a standard extrusion process. During the extrusion process, the antimicrobial agent, for example carvacrol, is added. The antimicrobial agent may be added at temperature high enough to allow for the chemicals to be embed while extruding the film, but also not so hot as to deactivate or minimize the efficacy of the antimicrobial agent. Since the temperature at which a particular agent may lose effectiveness as an antimicrobial may vary, so may the temperature at which the agent is added and the extrusion takes place. As a non-limiting example, carvacrol deactivates at about 101 °C (214 degrees Fahrenheit), so the extrusion temperature may be below about 101 °C (214 degrees Fahrenheit), and more preferably below about 93 °C (200 degrees Fahrenheit). In other instances, as described herein, the anti-microbial may be applied as a coating, which may allow for extrusion at higher temperatures.

As is known in the art, the process may be modified in a variety of ways without departing from the scope of the invention, such as applying the first adhesive to the membrane rather than the spacer sheet, applying the second adhesive to the release paper rather than the spacer sheet, using lasers to cut the layered material, layering and cutting the membrane and spacer before adding the release sheet and combinations thereof.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

The indefinite articles "a" and "an," as used herein in the specification and in the claims, unless clearly indicated to the contrary, should be understood to mean "at least one."

When used in this specification and the claims as an adverb rather than a preposition, "about" means "approximately" and comprises the stated value and every value within 10% of that value; in other words, "about 100%" includes 90% and 110% and every value in between.

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03.

## Claims

1. A puncture site patch (10; 110), comprising:
an elastomeric, self-sealing membrane (12; 112), wherein the elastomeric, self-sealing membrane (12; 112) further including an exterior face (11; 111) and an interior face (13; 113);
a spacer (14; 114) including an outer face (15; 115) and an inner face (17; 117),
wherein the outer face of the spacer (14; 114) is coupled to the interior face (13; 113) of the elastomeric, self-sealing membrane (12; 112);
wherein the spacer (14; 114) further includes an aperture (16; 116), the aperture (16; 116) forming a cavity (20; 120) defined by the interior face (13; 113) of the elastomeric, self-sealing membrane (12; 112), spacer (14; 114), and skin (90; 190) when applied to a user;
an adhesive film (26; 126) configured to adhere to skin (90; 190), the adhesive film (26; 126) on the inner face (17; 117) of the spacer (14; 114);
at least one elongated tab (24_{1, 2}; 124) configured to move between a first position and a second position;
wherein in the first position the at least one elongated tab (24_{1, 2}; 124) extends outward from a periphery of the spacer (14; 114); and
wherein in the second position the puncture site patch (10; 110) is folded so that the aperture (16; 116) is sealed closed by the at least one elongated tab (24_{1, 2}; 124),
**characterized in that**
an antimicrobial agent is incorporated into the elastomeric, self-sealing membrane (12; 112) during extrusion.

2. The puncture site patch (10; 110) of claim 1, wherein the at least one elongated tab (24_{1, 2}) is a first elongated tab (24₁) and the puncture site patch further includes a second elongated tab (24₂), wherein the first and second elongated tabs (24_{1, 2}) each extend outward from the periphery of the spacer (14; 114) and are disposed directly opposing each other.

3. The puncture site patch (10; 110) of claim 2, wherein in the second position the first and second elongated tabs (24_{1, 2}) overlap each other.

4. The puncture site patch (10; 110) according to any one of claims 1 to 3, further comprising an axis (A) running through a center of the aperture (16; 116) defining a first side of the spacer (14; 114) and a second side of the spacer (14; 114), and wherein in the second position the puncture site patch (10; 110) is folded along the axis such that the first side of the spacer (14; 114) and second side of the spacer (14; 114) overlap each other and the at least one elongated tab is folded over the overlapping first and second sides of the spacer (14; 114) to seal the first and second sides of the spacer (14; 114) closed.

5. The puncture site patch (10; 110) according to any one of claims 1 to 4, wherein in the second position the at least one elongated tab (24_{1, 2}; 124) is folded over the aperture (16; 116) to seal the cavity (20; 120) closed.

6. The puncture site patch (10; 110) according to any one of claims 1 to 5 further comprising a release paper (18; 118) in contact with and completely covering the adhesive film (26; 126), wherein the release paper (18; 118) is configured to be removed prior to application to skin (90; 190).

7. The puncture site patch (10; 110) according to any one of claims 1 to 6, wherein the at least one elongated tab (24_{1, 2}; 124) further included deadened adhesive.

8. The puncture site patch (10; 110) of according to any one of claims 1 to 7, wherein the elastomeric, self-sealing membrane (12; 112) is transparent.

9. The puncture site patch (10; 110) according to any one of claims 1 to 8, wherein the antimicrobial agent is a plurality of silver nanoparticles.

10. The puncture site patch according to any one of claims 1 to 9, wherein the antimicrobial agent is a carvacrol agent incorporated into the elastomeric, self-sealing, membrane (12; 112) at a concentration of about 0.01% to about 0.001%.

## Patentansprüche

1. Einstichstellenpflaster (10; 110), umfassend:
eine elastomere, selbstdichtende Membran (12; 112), wobei die elastomere, selbstdichtende Membran (12; 112) ferner eine Außenfläche (11; 111) und eine Innenfläche (13; 113) umfasst;
einen Abstandshalter (14; 114) mit einer Außenfläche (15; 115) und einer Innenfläche (17; 117),
wobei die Außenfläche des Abstandhalters (14; 114) mit der Innenfläche (13; 113) der elastomeren, selbstdichtenden Membran (12; 112) verbunden ist;
wobei der Abstandhalter (14; 114) ferner eine Öffnung (16; 116) umfasst, wobei die Öffnung (16; 116) einen Hohlraum (20; 120) bildet, der durch die Innenfläche (13; 113) der elastomeren, selbstdichtenden Membran (12; 112), dem Abstandhalter (14; 114) und Haut (90; 190) definiert ist, wenn sie an einem Benutzer angebracht ist;
einen Klebefilm (26; 126), der so konfiguriert ist, dass er an der Haut (90; 190) haftet, wobei sich der Klebefilm (26; 126) auf der Innenseite (17; 117) des Abstandhalters (14; 114) befindet;
mindestens eine längliche Lasche (24_{1, 2}; 124), die so konfiguriert ist, dass sie sich zwischen einer ersten Position und einer zweiten Position bewegt;
wobei sich in der ersten Position die mindestens eine längliche Lasche (24_{1, 2}; 124) von einem Umfang des Abstandhalters (14; 114) nach außen erstreckt; und
wobei in der zweiten Position das Einstichstellenpflaster (10; 110) so gefaltet ist, dass die Öffnung (16; 116) durch die mindestens eine längliche Lasche (24_{1, 2}; 124) versiegelt ist,
**dadurch gekennzeichnet, dass**
ein antimikrobielles Mittel während einer Extrusion in die elastomere, selbstdichtende Membran (12; 112) eingearbeitet wird.

2. Einstichstellenpflaster (10; 110) nach Anspruch 1, wobei die mindestens eine längliche Lasche (24_{1, 2}) eine erste längliche Lasche (24₁) ist und das Einstichstellenpflaster ferner eine zweite längliche Lasche (24₂) umfasst, wobei die erste und die zweite längliche Lasche (24_{1, 2}) sich jeweils vom Umfang des Abstandhalters (14; 114) nach außen erstrecken und einander direkt gegenüberliegend angeordnet sind.

3. Einstichstellenpflaster (10; 110) nach Anspruch 2, wobei in der zweiten Position sich die erste und die zweite längliche Lasche (24_{1, 2}) überlappen.

4. Einstichstellenpflaster (10; 110) nach einem der Ansprüche 1 bis 3, ferner umfassend eine Achse (A), die durch eine Mitte der Öffnung (16; 116) verläuft, die eine erste Seite des Abstandhalters (14; 114) und eine zweite Seite des Abstandhalters (14; 114) definiert, und wobei in der zweiten Position das Einstichstellenpflaster (10; 110) entlang der Achse so gefaltet ist, dass die erste Seite des Abstandhalters (14; 114) und die zweite Seite des Abstandhalters (14; 114) einander überlappen und die mindestens eine längliche Lasche über die überlappende erste und zweite Seite des Abstandhalters (14; 114) gefaltet ist, um die erste und zweite Seite des Abstandhalters (14; 114) zu versiegeln.

5. Einstichstellenpflaster (10; 110) nach einem der Ansprüche 1 bis 4, wobei in der zweiten Position die mindestens eine längliche Lasche (24_{1, 2}; 124) über die Öffnung (16; 116) gefaltet ist, um den Hohlraum (20; 120) zu versiegeln.

6. Einstichstellenpflaster (10; 110) nach einem der Ansprüche 1 bis 5, ferner umfassend ein Trennpapier (18; 118), das mit dem Klebefilm (26; 126) in Kontakt steht und diesen vollständig bedeckt, wobei das Trennpapier (18; 118) so konfiguriert ist, dass es vor dem Aufbringen auf die Haut (90; 190) entfernt werden kann.

7. Einstichstellenpflaster (10; 110) nach einem der Ansprüche 1 bis 6, wobei die mindestens eine längliche Lasche (24_{1, 2}; 124) ferner einen abgestumpften Klebstoff enthält.

8. Einstichstellenpflaster (10; 110) nach einem der Ansprüche 1 bis 7, wobei die elastomere, selbstdichtende Membran (12; 112) transparent ist.

9. Einstichstellenpflaster (10; 110) nach einem der Ansprüche 1 bis 8, wobei das antimikrobielle Mittel eine Vielzahl von Silbernanopartikeln ist.

10. Einstichstellenpflaster nach einem der Ansprüche 1 bis 9, wobei das antimikrobielle Mittel ein Carvacrol-Mittel ist, das in einer Konzentration von etwa 0,01 % bis etwa 0,001 % in die elastomere, selbstdichtende Membran (12; 112) eingearbeitet ist.

## Revendications

1. Timbre pour site de ponction (10 ; 110), comprenant :
une membrane auto-obturante élastomère (12; 112), dans lequel la membrane auto-obturante élastomère (12 ; 112) inclut en outre une face extérieure (11 ; 111) et une face intérieure (13 ; 113) ;
une entretoise (14 ; 114) incluant une face externe (15 ; 115) et une face interne (17 ; 117),
dans lequel la face externe de l'entretoise (14 ; 114) est accouplée à la face intérieure (13 ; 113) de la membrane auto-obturante élastomère (12 ; 112);
dans lequel l'entretoise (14 ; 114) inclut en outre une ouverture (16; 116), l'ouverture (16 ; 116) formant une cavité (20 ; 120) définie par la face intérieure (13 ; 113) de la membrane auto-obturante élastomère (12 ; 112), l'entretoise (14; 114), et la peau (90 ; 190) lorsqu'elle est appliquée sur un utilisateur ;
un film adhésif (26; 126) configuré pour adhérer à la peau (90 ; 190), le film adhésif (26 ; 126) étant situé sur la face interne (17 ; 117) de l'entretoise (14 ; 114) ;
au moins une languette allongée (24_{1, 2}; 124) configurée pour se déplacer entre une première position et une deuxième position ;
dans lequel, dans la première position, l'au moins une languette allongée (24_{1, 2}; 124) s'étend vers l'extérieur à partir d'une périphérie de l'entretoise (14 ; 114) ; et
dans lequel, dans la deuxième position, le timbre pour site de ponction (10 ; 110) est plié de sorte que l'ouverture (16; 116) est scellée par l'au moins une languette allongée (24_{1, 2} ; 124),
**caractérisé en ce que**
un agent antimicrobien est incorporé dans la membrane auto-obturante élastomère (12 ; 112) pendant l'extrusion.

2. Timbre pour site de ponction (10 ; 110) selon la revendication 1, dans lequel l'au moins une languette allongée (24_{1, 2}) est une première languette allongée (24₁) et le timbre pour site de ponction inclut en outre une deuxième languette allongée (24₂), dans lequel les première et deuxième languettes allongées (24_{1,2}) s'étendent chacune vers l'extérieur à partir de la périphérie de l'entretoise (14 ; 114) et sont disposées directement opposées l'une à l'autre.

3. Timbre pour site de ponction (10 ; 110) selon la revendication 2, dans lequel, dans la deuxième position, les première et deuxième languettes allongées (24_{1,2}) se chevauchent.

4. Timbre pour site de ponction (10 ; 110) selon l'une quelconque des revendications 1 à 3, comprenant en outre un axe (A) passant par un centre de l'ouverture (16 ; 116) définissant un premier côté de l'entretoise (14 ; 114) et un deuxième côté de l'entretoise (14; 114), et dans lequel, dans la deuxième position, le timbre pour site de ponction (10 ; 110) est plié le long de l'axe de sorte que le premier côté de l'entretoise (14 ; 114) et le deuxième côté de l'entretoise (14; 114) se chevauchent et l'au moins une languette allongée est pliée sur les premier et deuxième côtés chevauchants de l'entretoise (14 ; 114) pour sceller les premier et deuxième côtés de l'entretoise (14 ; 114).

5. Timbre de site de ponction (10 ; 110) selon l'une quelconque des revendications 1 à 4, dans lequel, dans la deuxième position, l'au moins une languette allongée (24_{1,2}; 124) est pliée sur l'ouverture (16 ; 116) pour sceller la cavité (20 ; 120).

6. Timbre pour site de ponction (10 ; 110) selon l'une quelconque des revendications 1 à 5 comprenant en outre un papier antiadhésif (18 ; 118) en contact avec le film adhésif (26 ; 126) et le recouvrant complètement, dans lequel le papier antiadhésif (18 ; 118) est configuré pour être retiré avant l'application sur la peau (90 ; 190).

7. Timbre pour site de ponction (10 ; 110) selon l'une quelconque des revendications 1 à 6, dans lequel l'au moins une languette allongée (24_{1, 2} ; 124) inclut en outre un adhésif atténué.

8. Timbre pour site de ponction (10 ; 110) selon l'une quelconque des revendications 1 à 7, dans lequel la membrane auto-obturante élastomère (12 ; 112) est transparente.

9. Timbre pour site de ponction (10 ; 110) selon l'une quelconque des revendications 1 à 8, dans lequel l'agent antimicrobien est une pluralité de nanoparticules d'argent.

10. Timbre pour site de ponction selon l'une quelconque des revendications 1 à 9, dans lequel l'agent antimicrobien est un agent carvacrol incorporé dans la membrane auto-obturante élastomère (12 ; 112) à une concentration d'environ 0,01 % à environ 0,001 %.
